# EUROPEAN PATENT APPLICATION

(11) **EP 3 542 865 A1**
(43) Date of publication of application: **25.09.2019**
(21) Application number: 18163029.4
(22) Date of filing: 21.03.2018
(51) Int. Cl.: A61Q 5/12, A61K 8/37, A61K 8/41, A61K 8/49, A61Q 5/06, A61K 8/81, A61K 8/86, A61K 8/891, A61K 8/892, A61K 8/92, A61K 8/9783

(54) **HAIR CONDITIONING COMPOSITION**

(71) Applicant: Kao Germany GmbH, 64297 Darmstadt (DE)
(72) Inventor: Hoffmann, Martin, 64297 Darmstadt Hessen (DE); Entzminger, Anne, 64297 Darmstadt (DE)

(57) **Abstract**

The present invention relates to an aqueous composition for treating hair comprising one or more non-volatile oil(s) and one or more cationic polymers having certain cationic charge density as deposition aid to the non-volatile oils. The novel composition provides hair long lasting conditioning, especially improves combability, shine and suppleness of hair which is held up to 20 hair washes by enhanced deposition of the oils onto hair. It is found out that the non-volatile oils are not washed off from hair with subsequent washes and detected on the hair up to 20 hair washes.

## Description

The present invention relates to an aqueous composition for treating hair comprising non-volatile one or more oil(s) and one or more cationic polymers having certain cationic charge density as deposition aid to the non-volatile oils. The novel composition provides hair long lasting conditioning, especially improves combability, shine and suppleness of hair which is held up to 20 hair washes by enhanced deposition of the oils onto hair. It is found out that the non-volatile oils are not washed off from hair with subsequent washes and detected on the hair up to 20 hair washes.

Hair conditioners are widely used for improving hair combability and suppleness among other properties which are as well improved and/or given. Consumers use the conditioners after each shampooing as hair combability and suppleness is usually reduced after cleansing the hair surface. Such process takes long time under shower and, therefore, there is a great need for hair conditioners which provides hair improved long lasting combability and suppleness. In other words, the consumers do not need to use a conditioning composition after each hair shampooing for improving combability and suppleness for certain number of hair washes.

The above mentioned problems have been subject of numerous research projects. The one approach has been improving hair conditioning properties of cleansing compositions so that there is no need for using of an additional composition only for making hair better combable and supple. In this way there have been many product initiatives on the market in form of so called two-in-one shampoo compositions.

Oils liquid at ambient conditions have been known for their conditioning effect on hair in terms of softness, shine and combability enhancements. It is also known that after one shampooing cycle these compounds are completely washed off from hair and therewith no further conditioning benefits are left on the hair. There is a great need for improving their deposition on the hair so that hair condition is stayed improved for a long period of time, i.e. several hair washes.

DE 10 2013225898 discloses compositions for long lasting hair conditioning comprising cationic polymer with a relatively high charge density. It has been observed that compositions disclosed therein do not really provide the long lasting conditioning effects because of several reasons which are made clear below.

WO 2016/206740 A1 discloses compositions for treating hair comprising high charge density and aminated silicones for long lasting hair conditioning. The document is silent on the action of the high charge density polymers as deposition aid for oils.

The inventors of the present invention have surprisingly find out that cationic quaternary ammonium polymers having high cationic charge density acts as a deposition aid to oils which are liquid at 20°C and at atmospheric pressure in an aqueous composition which results in further improvement of hair status in terms of combability, suppleness, and shine which may hold with repeated hair washes up to 20 wash cycles. It has further been observed that the oils deposited onto hair stay on the hair for several washes.

Accordingly, the object of the present invention is an aqueous composition for human hair comprising one or more non-volatile oils liquid at 20°C and at atmospheric pressure and one or more cationic quaternary ammonium polymers having a cationic charge density of 3.0 mEq/g or more as deposition aid to the one or more non-volatile oils.

Further object of the present invention is the method of depositing non-volatile oils liquid at 20°C and at atmospheric pressure wherein a composition according to present invention as outlined above is applied onto hair and rinsed off from hair after leaving on the hair for a period 1 to 30 min at ambient temperature.

Still further object of the present invention, method of depositing non-volatile oils onto hair which are liquid at 20°C and at atmospheric pressure wherein a composition according to present invention as outlined above is applied onto hair and rinsed off from hair after leaving on the hair for a period 1 to 30 min wherein the non-volatile oil(s) are not washed off from hair completely with single shampooing, preferably not washed off from hair completely with 1 to 5 shampooing cycles.

The aqueous composition of the present invention comprises one or more cationic quaternary ammonium polymers having a charge density of 3 mEq/g or more, preferably 3.5 to 8 mEq/g, more preferably 4 to 7.5 mEq/g, most preferably 4.5 to 7.0 mEq/g and in particular 4.5 to 6.5 mEq/g. In a preferred embodiment of the present invention the aqueous composition comprises only one cationic quaternary ammonium polymer with the above defined cationic charge density ranges.

Suitable non-limiting cationic quaternary ammonium polymers for the purpose of the present invention are Polyquaternium-37, Polyquaternium-6 and Polyquaternium-16.

The preferred cationic polymers are Polyquaternium-37 and Polyquaternium-16. The particularly preferred cationic polymer is Polyquaternium-37.

Total cationic quaternary ammonium polymer concentration having the above described cationic charge density is in the range of 0.1 to 5%, preferably 0.2 to 4%, more preferably 0.25 to 3% and most preferably 0.3 to 2.5% by weight, calculated to the total composition.

The aqueous composition of the present invention comprises one or more non-volatile oil liquid at 20°C and at atmospheric pressure. The non-volatile oils are suitably selected from non-volatile silicone oils, natural and synthetic oils, fatty alcohol ethers (dialkyl ethers) and fatty acid fatty alcohol esters. Among silicone oils mention is made to dimethicone, dimethiconol, polydimethylsiloxane (DC fluid ranges from Dow Corning), arylated silicones such as phenyl methicone, phenyl trimethicone, diphenyl dimethicone, diphenylsiloxy phenyl trimethicone, tetramethyl tetraphenyl trisiloxane, triphenyl trimethicone, and trimethyl pentaphenyl trisiloxane.

The term non-volatile oil and silicone oil means that the oil does not have any measurable vapor pressure at room temperature, 20°C.

Suitable non-limiting examples to natural plant oils are such as olive oil, almond oil, avocado oil, ricinus oil, avocado oil, coconut oil, palm oil, sesame oil, peanut oil, whale oil, sunflower oil, peach kernel oil, wheat germ oil, macadamia nut oil, night primrose oil, jojoba oil, soya oil, Passiflora oil, Black cumin oil, Borage oils, Evening Primrose oil, Grapeseed oil, Hempseed oil, Kukui nut oil, Rosehip oil, Safflower oil, Walnut oil.

Suitable non-limiting examples to fatty alcohol fatty acid esters are such as isopropyl myristate, palmitate, stearate and isostearate, oleyl oleate, isocetyl stearate, hexyl laurate, dibutyl adipate, dioctyl adipate, myristyl myristate and oleyl erucate.

Suitable non-limiting examples to fatty alchol ethers (dialkyl ethers) are such as dimyristyl ether, dicetyl ether and dicaprylyl ether.

The composition comprises one or more oil at a total concentration in the range of 0.01 to 10%, preferably 0.05 to 7.5%, more preferably 0.1 to 5 and most preferably 0.1 ta 3% by weight calculated to total composition.

The aqueous composition of the present invention preferably comprises one or more surfactants selected from nonionic and cationic ones.

Suitable nonionic surfactants are fatty alcohol ethoxylates of the following general structure

R₁ (OCH₂CH₂)ₙ OH

wherein R₁ is straight or branched, saturated or unsaturated alkyl chain which may be synthetic or natural with a C chain length in the range of 8 to 40, preferably 9 to 30 and more preferably 9 to 24 and n is a number in the range of 5 to 40, preferably 9 to 30.

Non-limiting suitable examples of the fatty alcohol ethoxylates are C9-11 Pareth-6, C9-11 Pareth-8, C9-15 Pareth-8, C11-13 Pareth-9, C11-13 Pareth-10, C11-15 Pareth-5, C11-15 Pareth-7, C11-15 Pareth-9, C11-15 Pareth-12, C11-15 Pareth-15, C11-15 Pareth-20, C11-15 Pareth-30, C11-15 Pareth-40, C11-21 Pareth-10, C12-13 Pareth-5, C12-13 Pareth-6, C12-13 Pareth-7, C12-13 Pareth-9, C12-13 Pareth-10, C12-13 Pareth-15, C12-13 Pareth-23, C12-14 Pareth-5, C12-14 Pareth-7, C12-14 Pareth-9, C12-14 Pareth-11, C12-14 Pareth-12, C12-15 Pareth-5, C12-15 Pareth-7, C12-15 Pareth-9, C12-15 Pareth-10, C12-15 Pareth-11, C12-15 Pareth-12, C12-16 Pareth-5, C12-16 Pareth-7, C12-16 Pareth-9, C13-15 Pareth-21, C14-15 Pareth-7, C14-15 Pareth-8, C14-15 Pareth-11, C14-15 Pareth-12, C14-15 Pareth-13, C20-22 Pareth-30, C20-40 Pareth-10, C20-40 Pareth-24, C20-40 Pareth-40, C20-40 Pareth-95, C22-24 Pareth-33, Beheneth-5, Beheneth-10, Beheneth-15, Beheneth-20, Beheneth-25, Beheneth-30, Ceteareth-5, Ceteareth-6, Ceteareth-7, Ceteareth-10, Ceteareth-11, Ceteareth-12, Ceteareth-15, Ceteareth-20, Ceteareth-25, Ceteareth-30, Ceteareth-35, Ceteareth-40, Laureth-5, Laureth-10, Laureth-15, Laureth-20, Laureth-25, Laureth-30, Laureth-40, Myreth-5, Myreth-10, Ceteth-5, Ceteth-10, Ceteth-15, Ceteth-20, Ceteth-25, Ceteth-30, Ceteth-40, Oleth-5, Oleth-10, Oleth-15, Oleth-20, Oleth-25, Oleth-30, Oleth-40, Steareth-5, Steareth-10, Steareth-15, Steareth-20, Steareth-25, Steareth-30, Steareth-35, and Steareth-40. They may also be comprised in the compositions as a mixture of more than one surfactant. Further suitable nonionic surfactant is polypropylene glycol ether of fatty alcohols according to general structure

R₂(OCH₂(CH₃)CH₂)ₙOH

wherein R₂ is straight or branched, saturated or unsaturated fatty alcohol which may be synthetic or natural with a C chain length in the range of 8 to 40, preferably 9 to 30 and more preferably 9 to 24 and n is a number in the range of 1 to 40, preferably 3 to 30.

Suitable non-limiting examples are PPG-3 Caprylyl ether, PPG-5 Caprylyl ether, PPG-10 Caprylyl ether, PPG-10 Cetyl ether, PPG-20 Cetyl ether, PPG-28 Cetyl ether, PPG-30 Cetyl ether, PPG-7 Lauryl ether, PPG-10 Lauryl ether, PPG-10 Oleyl ether, PPG-20 Oleyl ether, PPG-23 Oleyl ether, PPG-30 Oleyl ether, PPG-11 Stearyl ether and PPG-15 Stearyl ether.

Further suitable nonionic surfactants are polyethylene glycol fatty acid esters of the following general structure

R₃ C(O) (OCH₂CH₂)ₙ OH

wherein R₃ is straight or branched, saturated or unsaturated alkyl group which may be synthetic or natural with a C chain length in the range of 7 to 39, preferably 9 to 29 and more preferably 9 to 23 and n is a number in the range of 5 to 40, preferably 9 to 30.

Suitable non-limiting examples are PEG-8 Behenate, PEG-8 Caprate, PEG-8 Caprylate, PEG-5 Cocoate, PEG-8 Cocoate, PEG-9 Cocoate, PEG-10 Cocoate, PEG-15 Cocoate, PEG-6 Isopalmitate, PEG-6 Isostearate, PEG-8 Isostearate, PEG-9 Isostearate, PEG-10 Isostearate, PEG-12 Isostearate, PEG-20 Isostearate, PEG-30 Isostearate, PEG-40 Isostearate, PEG-6 Laurate, PEG-8 Laurate, PEG-9 Laurate, PEG-10 Laurate, PEG-12 Laurate, PEG-14 Laurate, PEG-20 Laurate, PEG-30 Laurate, PEG-8 Myristate, PEG-20 Myristate, PEG-5 Oleate, PEG-6 Oleate, PEG-7 Oleate, PEG-8 Oleate, PEG-9 Oleate, PEG-10 Oleate, PEG-11 Oleate, PEG-12 Oleate, PEG-15 Oleate, PEG-20 Oleate, PEG-30 Oleate, PEG-32 Oleate, PEG-6 Palmitate, PEG-18 Palmitate, PEG-20 Palmitate, PEG-5 Stearate, PEG-6 Stearate, PEG-7 Stearate, PEG-8 Stearate, PEG-9 Stearate, PEG-10 Stearate, PEG-12 Stearate, PEG-14 Stearate, PEG-15 Stearate, PEG-20 Stearate, PEG-25 Stearate, PEG-30 Stearate, PEG-35 Stearate and PEG-40 Stearate.

Further suitable nonionic surfactants are polypropylene glycol fatty acid esters of the following general structure

R₄C(O)(OCH₂(CH₃)CH₂)ₙOH

wherein R₄ is straight or branched, saturated or unsaturated alkyl group which may be synthetic or natural with a C chain length in the range of 7 to 39, preferably 9 to 29 and more preferably 9 to 23 and n is a number in the range of 1 to 40, preferably 9 to 30.

Suitable non-limiting examples are PPG-15 Isostearate, PPG-9 Laurate, PPG-26 Oleate and PPG-36 Oleate.

Further nonionic suitable surfactants are polyethylene glycol and polypropylene glycol ether of fatty alcohols of the following general structure

R₅(OCH₂(CH₃)CH₂)ₙ₁(OCH₂CH₂)ₙ₂OH

wherein R₅ is straight or branched, saturated or unsaturated alkyl group which may be synthetic or natural with a C chain length in the range of 7 to 39, preferably 9 to 29 and more preferably 9 to 23 and n1 and n2 may be the same or different and are a number in the range of 1 to 40.

Suitable non-limiting examples are PPG-2 Ceteareth-9, PPG-4 Ceteareth-12, PPG-4 Ceteareth-20, PPG-2 C9 - 11 Pareth-5, PPG-2 C9 - 11 Pareth-7, PPG-2 C9 - 11 Pareth-8, PPG-2 C9 - 11 Pareth-11, PPG-2 C12 - 13 Pareth-8, PPG-2 C12 - 15 Pareth-6, PPG-4 C 13- 15 Pareth-15, PPG-5 C9 - 15 Pareth-6, PPG-6 C9 - 11 Pareth-5, PPG-6 C12 - 15 Pareth-12, PPG-6 C12 - 18 Pareth-11, PPG-1 Deceth-4, PPG-1 Deceth-5, PPG-1 Deceth-6, PPG-1 Deceth-7, PPG-2 Deceth-3, PPG-2 Deceth-7, PPG-2 Deceth-8, PPG-2 Deceth-10, PPG-2 Deceth-15, PPG-2 Deceth-20, PPG-2 Deceth-30, PPG-2 Deceth-40, PPG-4 Deceth-4, PPG-4 Deceth-6, PPG-4 Deceth-6, PPG-6 Deceth-4, PPG-6 Deceth-9, PPG-8 Deceth-6, PPG-14 Deceth-6, PPG-2 Laureth-5, PPG-2 Laureth-8, PPG-2 Laureth-12, PPG-3 Laureth-8, PPG-3 Laureth-9, PPG-3 Laureth-10, PPG-3 Laureth-12, PPG-4 Laureth-2, PPG-4 Laureth-5, PPG-4 Laureth-7, PPG-4 Laurreth-15, PPG-5 Laureth-5, PPG-5 Laureth-3, PPG-20 Laureth-12, PPG-25 Laureth-25, PPG-3 Myreth-3, PPG-3 Myreth-11, PPG-9 Steareth-3, PPG-23 Steareth-34, PPG-30 Steareth-4, PPG-34 Steareth-3, and PPG-38 Steareth-6.

Further suitable nonionic surfactants are ethoxylated triglycerides. Well known and commonly used ones are ethoxylated castor oil such as PEG-40 hydrogenated castor oil or and PEG-60 hydrogenated castor oil.

Further suitable surfactants are those of glucamides such as lauroyl/myristoyl methyl glucamide and cocoyl methyl glucamide.

Total concentration of non-ionic surfactants is in the range of 0.1 to 5%, preferably 0.2 to 4%, more preferably 0.25 to 3% and most preferably 0.3 to 2.5% by weight, calculated to the total composition.

The aqueous composition of the present invention may comprise quaternary ammonium surfactant of the general structure

R₁₁ R₁₂ R₁₃ R₁₄ N

wherein R₁₁ is an alky chain having C length of 8 to 30 which may be saturated or unsaturated, straight or branched, R₁₂ an alky chain having C length of 1 to 30 which may be saturated or unsaturated, straight or branched, R₁₁ and R₁₂ additionally may take the structures of

R₁₅ C(O) O (CH2) n or R₁₅ C(O) NH (CH2)n

wherein R15 is an alkyl chain with a C length of 7 to 29 which may be saturated or unsaturated, straight or branched and n is a number between 1 and 4, R₁₃ and R₁₄ is an alkyl chain with a C length of 1 to 4 which may be straight or branched (only for C3 and C4), wherein all alkyl chains may include one or more substituents such as hydroxyl or (poly)ethoxy groups.

Suitable nonlimiting examples are cetyl trimethyl ammonium chloride, stearyl trimonium chloride, dipalmitoyl dimonium chloride, distearyl dimethyl ammonium chloride, stearamidopropyl trimethyl ammonium chloride, dioleoylethyl dimethyl ammonium methosulfate, dioleoylethyl hydroxyethylmonium methosulfate, behenyl trimethyl ammonium chloride, and/or their mixtures.

Quaternary ammonium surfactants are comprised in the compositions of the present invention at a total concentration in the range of 0.1 to 5%, preferably 0.2 to 4%, more preferably 0.25 to 3% and most preferably 0.3 to 2.5% by weight, calculated to the total composition.

The composition of the present invention may comprise one or more fatty alcohols and may be in a form of emulsion. Suitable non-limiting examples are according to general structure

R₆-OH

wherein R₆ is straight or branched, saturated or unsaturated alkyl chain which may be synthetic or natural with a C chain length in the range of 12 to 24 which may as well be substituted with one or more groups on the alkyl chain.

Non limiting suitable examples are lauryl alcohol, myristyl alcohol, cetyl alcohol, oleyl alcohol, stearyl alcohol, behenyl alcohol and their mixtures. As a mixed fatty alcohol the mostly used one is the cetearyl alcohol as well the preferred fatty alcohol in the compositions of the present invention.

The total concentration of one or more fatty alcohols is in the range of 0.1 to 20%, preferably 0.5 to 15%, more preferably 1 to 10% and the most preferably 2 to 7.5% by weight calculated to the total composition.

The aqueous composition of the present invention may comprise one or more organic solvents. Suitable ones are ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, polypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are ethanol, isopropanol, benzylalcohol and polypropylene glycols. Concentration of organic solvents should not exceed 20% by weight, preferably in the range of 0.1 to 15 %, more preferably 0.1 to 10% by weight and most preferably 0.1 to 7.5% by weight calculated to total composition. It should be noted that concentration of one or more organic solvents is very much dependent on the type of preparation i.e. a solution can contain higher concentration of organic solvent than a gel or emulsion composition.
Further in an embodiment of the present invention, the aqueous composition of the present invention comprises at least one hair direct dye selected from cationic and neutral nitro dyes.

Any cationic direct dye is in principal suitable for the compositions. Non-limiting suitable examples are Basic Blue 6, Basic Blue 7, Basic Blue 9, Basic Blue 26, Basic Blue 41, Basic Blue 99, Basic Blue 124, Basic Brown 4, Basic Brown 7, Basic Brown 16, Basic Brown 17, Basic Green 1, Basic Orange 31, Basic Red 2, Basic Red 12, Basic Red 22, Basic Red 51, Basic Red 76, Basic Violet 2, Basic Violet 3, Basic Violet 10, Basic Violet 14, Basic Violet 57 and Basic Yellow 87.

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Total concentration of one or more direct dyes in total is in the range of 0.001 to 1.0% by weight, preferably 0.01 to 0.8% more preferably 0.05 to 0.75% and most preferably 0.1 to 0.5% by weight calculated to total composition.

The aqueous composition of the present invention may further comprise one or more UV filters which may be selected from water soluble ones as well as oils soluble ones. The oil soluble UV filter are more preferred ones as they show no interaction with the cationic quaternary ammonium polymers. Non-limiting examples are 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, and/or polysilicone-15.

The total UV filter concentration may be in the range of 0.01 to 2.5 % by weight calculated to the total composition.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates.

The composition of the present invention may further comprise natural plant extracts. Within the meaning of the present invention the extracts are liquid extracts and prepared by mixing plant parts such as leaves, fruits, blossoms and roots with a solvent such as water, alcohol, propyleneglycol or mixture of more than one solvent and incubating for certain period of time and filtrating the undissolved plant parts. Suitable aqueous (e.g. steam-distilled) alcoholic or hydro-alcoholic plant extracts known **per se** are in particular aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, the various "Extrapon®" products, "Herbasol^{R} ", "Sedaplant^{R}" and "Hexaplant^{R}". Extracts and the preparation thereof are also described in "Hagers Handbuch der pharmazeutischen Praxis", 4th Ed. Preferred plant extracts are prepared from Vitis vinifera, Malus domestica, Camelia sinensis, Juglans regia Ribes Uva-Crispa, Ribes nigrum, Ribes rubrum and Punica granatum. The above mentioned extracts may also be available in the powder form and such are also suitable within the meaning of the present invention.

The natural plant extracts are included into the compositions at a concentration of 0.001 to 1.0%, preferably 0.005 to 0.75%, more preferably 0.01 to 0.6% and most preferably 0.05 to 0.5% by weight, calculated to total composition based on dry matter of the extract.

Further in an embodiment of the present invention, compositions comprise one or more ubiquinone of the following general structure 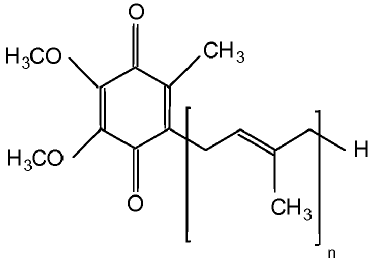 wherein n is a number between 1 and 10. It should be noted that the compositions of the present invention can certainly comprise more than one ubiquinone. Preferred ubiquinones are the ones where n is a number between 6 and 10 and especially preferred is Ubiquinone 50 where n is 10, also known as Coenzyme Q10.

Concentration ubiquinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

The pH of the compositions according to the present invention is suitably between 3.0 and 7.0 and preferably in the range of 3.5 to 6.5, more preferably 4.0 to 5.5 and most preferably 4.0 to 5.

In principal pH of the compositions can be adjusted with any organic and/or inorganic acids or their mixture. Some of them to mention are phosphoric acid, hydrochloric acid as the inorganic ones and to the organic acids the well-known citric acid and lactic acid, glycolic acid, glyoxylic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid.

Conditioning compositions of the present invention can comprise additionally any compound customarily found in conditioning compositions such as chelating agents, preservatives and fragrance.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

Viscosity of the conditioning composition may be adjusted according to the application form and generally should not be more than 50,000 mPa.s at 20°C measured with Brookfield Rheometer at a shear rate of 5 sec⁻¹.

Thickening agents, especially the nonionic thickening polymers, may additionally be comprised into the compositions of the present invention. Suitable non-limiting examples are cellulose derivatives such as hydroxyethyl cellulose, methyl cellulose, hydroxymethyl cellulose, hydroxypropyl cellulose, guar gum and its derivatives, and cognac mannan and derivatives. Thickeners may be included at a concentration of 0.05 to 2.5% by weight calculated to total composition. Concentration of thickener is very much dependent on the thickener itself and also the preparation such as pH value of the composition etc. and therefore should be selected depending on the desired viscosity of the composition.

The aqueous composition of the present invention may be in the form of solution, gel, dispersion and emulsion. These preparations may be provided to the consumers as they are confectioned in a suitable packaging which allows easy release of the products and distribution onto hair as well as they may be provided in a pressurized container. In case pressurized container is used as the vessel carrying the composition, it additionally comprises a pressurizing gas which may be selected form the any known ones such as alkanes , butane, isobutene, dimethyl ether, pressurized air, nitrogen and carbon dioxide.

The following examples are to illustrate the invention but not to limit it.

### Example 1

The following compositions were prepared for the comparative tests.

| | A** | B | C | D** | E** |
|---|---|---|---|---|---|
| Polyquaternium-37 | 0.74 | - | - | 0.37 | 0.37 |
| Cyclopentasiloxane | - | 99.26 | 99.26 | - | - |
| Dimethicone/Dimethiconol | - | 0.74 | - | 0.37* | - |
| Trimethyl pentaphenyl trisiloxane | - | - | 0.74 | - | 0.37 |
| Water | 99.26 | - | - | 99.26 | 99.26 |

| | | | | | |
|---|---|---|---|---|---|
| *:Dimethiconol concentration **: pH is adjusted to the range 4.0 to 5.0 with citric acid/sodium hydroxide. The compositions D and E are within the scope of the claim 1. | | | | | |

Deposition of the oil compounds from the above compositions was determined on twice bleached Level 7 Caucasian hair tresses. 1 g of each composition was applied on at least two hair tresses separately each weighing approximately 2 g and left for 10 min at ambient temperature and rinsed off thoroughly with tap water at around 40°C and the streaks were dried.

The suppleness of the hair was measured with a zwick machine and expressed as the force (in milliNewton - mN) to pull off a streak through the rods of the machine. The machine is commercially available from Zwick/Roell Company and includes a manual. It was used according to its manual. Briefly, the streak was places between the metal rods placed offset in a given distance horizontally (2 cm) and vertically (8 cm) 5 in total and the force needed to pull the streaks through the metal rods at a given rate (5 cm/min) was measured. All values reported in the Table below are average of at least 6 readings.

In order to test the long lastingness of hair suppleness, the hair streaks were washed 5 times first and the suppleness was measured again and after additional wash the measurements were repeated. For washing hair streaks, 5% by weight Sodium lauryl ether sulfate solution in water at pH 5.0 was used.

**Table I: Suppleness of hair and its long lasting as measured with a zwick machine and expressed as the force to pull through the hair streak in mN.**

| Composition | 0 wash | 5 washes | 10 washes | 15 washes | 20 washes |
|---|---|---|---|---|---|
| A | 550 | 560 | 560 | 580 | 600 |
| B | 540 | 1030 | 1140 | 1420 | 1570 |
| C | 530 | 840 | 1000 | 1160 | 1300 |
| D | 360 | 450 | 490 | 500 | 480 |
| E | 460 | 400 | 440 | 450 | 480 |

From the above result it is clear that the effect of Polyquaternium-37 is long lasting. However, the effects of silicone oils is not long lasting at all, as the force needed to pull through the streak is increased with number of hair washes. As soon as the oils are dispersed in Polyquesternium-37 comprising aqueous composition, the suppleness effect becomes higher and it is long lasting. The force needed to pull the streak is after 20 washes still lower than the force measured with a streak treated with Polyquaternium -37 prior to washing.

It should be noted that the lower the value, the higher the suppleness of the hair.

The above results prove beyond any doubt the improved suppleness of the hair treated with the aqueous composition of the present invention.
Additionally, the deposited amount of dimethiconol and Trimethyl pentaphenyl trisiloxane was analysed by HPLC. Therefore, 2 g treated hair was cut into small pieces and placed into 30 ml tetrahydrofuran and the mixture was stirred for 5 min and afterwards placed in a ultrasonic water bath operated in 120 Watt for 2 min. The solution was filtered and analyzed by HPLC.

HPLC analysis method is as follows:
Phenomenex, Kinetex 2,6 u C18 (10 x 4.6 mm) column was used. The solvent was tetrahydrofuran and flow rate was 0.35 ml/min at 35°C. Injection volume was 20 µl and the detection was Diode Array Detector 220 nm. Retention time of dimethiconol and Trimethyl pentaphenyl trisiloxane was around 2.6 min. The quantitative analysis was made by measuring the area under the peak electronically and by comparing to the calibration curve made under similar conditions.

The results are presented in the Table II below.

**Table II: Deposited concentrations of Dimethiconol and Trimethyl pentaphenyl trisiloxane on hair.**

| Wash | | A | B | C | D | E |
|---|---|---|---|---|---|---|
| 0 | Dimethiconol | - | 100 | | 100 | |
| 0 | Trimethyl pentaphenyl trisiloxane | - | | 100 | | 100 |
| | | | | | | |
| 1 | Dimethiconol | - | 10 | | 52 | |
| 1 | Trimethyl pentaphenyl trisiloxane | - | | 10 | | 58 |
| | | | | | | |
| 5 | Dimethiconol | - | 1 | | 43 | |
| 5 | Trimethyl pentaphenyl trisiloxane | - | | 1 | | 46 |

In the above Table the results are presented as the % of prior to 1^{st} washing, immediately after application of the conditioners. From the above results it is clear that the dimethiconol and Trimethyl pentaphenyl trisiloxane remain on the hair longer than 5 hair washes when applied onto hair in the presence of Polyquaternium-37 as the cationic deposition polymer.

The similar results were observed with other high charge density cationic polymers such as Polyquaternium-6 and Polyquaternium-16.

The following examples fall within the scope of the present invention.

### Example 2

| | % by weight |
|---|---|
| Polyquaternium-37 | 0.80 |
| C12-13 Pareth-9 | 0.50 |
| Cetrimonium chloride | 0.25 |
| Dimethicone/Dimethiconol | 1.00 |
| Hydroxyethylcellulose | 0.50 |
| Lactic acid | q.s. to pH 4.2 |
| Preservative | q.s. |
| Water | to 100 |

### Example 3

| | % by weight |
|---|---|
| Polyquaternium-37 | 0.80 |
| Cetrimonium chloride | 0.50 |
| Trimethyl pentaphenyl trisiloxane | 0.50 |
| Hydroxypropyl guar | 0.50 |
| Lactic acid | q.s. to pH 4.2 |
| Preservative | q.s. |
| Water | to 100 |

### Example 4

| | % by weight |
|---|---|
| Polyquaternium-37 | 0.80 |
| C12-13 Pareth-9 | 0.50 |
| Trimethyl pentaphenyl trisiloxane | 0.50 |
| Cetrimonium chloride | 0.50 |
| Basic Red 51 | 0.20 |
| Basic Orange 31 | 0.02 |
| Basic Yellow 87 | 0.08 |
| Citric acid | q.s. to pH 4.8 |
| Preservative | q.s. |
| Water | to 100 |

### Example 5

| | % by weight |
|---|---|
| Polyquaternium-37 | 0.80 |
| Behentrimonium chloride | 0.85 |
| Dimethicone/Dimethiconol | 0.50 |
| Benzophenone-3 | 0.25 |
| Basic Red 51 | 0.20 |
| Basic Orange 31 | 0.02 |
| Basic Yellow 87 | 0.08 |
| Citric acid | q.s. to pH 4.8 |
| Preservative | q.s. |
| Water | to 100 |

### Example 6

| | % by weight |
|---|---|
| Polyquaternium-37 | 0.80 |
| C12-13 Pareth-9 | 0.85 |
| Olive oil | 0.50 |
| Steartrimonium chloride | 0.20 |
| Benzophenone-3 | 0.25 |
| Ethylhexylmethoxycinnamate | 0.10 |
| Basic Red 51 | 0.20 |
| Basic Orange 31 | 0.02 |
| HC red no 3 | 0.08 |
| Citric acid | q.s. to pH 5.0 |
| Preservative | q.s. |
| Water | to 100 |

### Example 7

| | % by weight |
|---|---|
| Polyquaternium-37 | 0.80 |
| C12-13 Pareth-9 | 0.85 |
| Isopropyl myristate | 0.50 |
| Behentrimonum chloride | 0.10 |
| Benzophenone-3 | 0.25 |
| Ethylhexyl methoxycinnamate | 0.10 |
| Hydroxypropyl guar gum | 0.25 |
| Basic Red 51 | 0.20 |
| Basic Orange 31 | 0.02 |
| HC red no 3 | 0.08 |
| Citric acid | q.s. to pH 4.8 |
| Preservative | q.s. |
| Water | to 100 |

### Example 8

| | % by weight |
|---|---|
| Polyquaternium-37 | 0.80 |
| Ceteareth 20 | 1.25 |
| Cetearyl alcohol | 5.00 |
| Behenamidopropyl trimonium chloride | 0.15 |
| Isopropyl palmitate | 0.50 |
| Dimethicone/Dimethiconol | 0.85 |
| Ethylhexyl methoxy cinnamate | 0.10 |
| Lactic acid | q.s. to pH 4.9 |
| Preservative | q.s. |
| Water | to 100 |

### Example 9

| | % by weight |
|---|---|
| Polyquaternium-37 | 0.80 |
| Ceteareth 30 | 1.25 |
| Cetearyl alcohol | 2.00 |
| Behenyl alcohol | 2.80 |
| Dimethicone/Dimethiconol | 0.50 |
| Almond oil | 0.85 |
| Vitis vinifera extract | 0.10 (dry matter) |
| Lactic acid | q.s. to pH 4.5 |
| Preservative | q.s. |
| Water | to 100 |

### Example 10

| | % by weight |
|---|---|
| Polyquaternium-37 | 0.80 |
| Ceteareth 30 | 1.25 |
| Cetearyl alcohol | 2.00 |
| Behenyl alcohol | 2.80 |
| Basic Red 51 | 0.20 |
| Basic Orange 37 | 0.02 |
| HC red no 3 | 0.08 |
| Basic yellow 87 | 0.03 |
| HC Yellow 2 | 0.03 |
| Trimethyl pentaphenyl trisiloxane | 0.85 |
| Almond oil | 0.10 |
| Vitis vinifera extract | 0.10 (dry matter) |
| Lactic acid | q.s. to pH 4.5 |
| Preservative | q.s. |
| Water | to 100 |

### Example 11

| | % by weight |
|---|---|
| Polyquaternium-6 | 0.80 |
| Ceteareth 30 | 1.25 |
| Cetearyl alcohol | 2.00 |
| Behenyl alcohol | 2.80 |
| Dimethicone/Dimethiconol | 0.50 |
| Almond oil | 0.85 |
| Vitis vinifera extract | 0.10 (dry matter) |
| Lactic acid | q.s. to pH 4.5 |
| Preservative | q.s. |
| Water | to 100 |

### Example 12

| | % by weight |
|---|---|
| Polyquaternium-16 | 0.80 |
| Ceteareth 30 | 1.25 |
| Cetearyl alcohol | 2.00 |
| Behenyl alcohol | 2.80 |
| Basic Red 51 | 0.20 |
| Basic Orange 37 | 0.02 |
| HC red no 3 | 0.08 |
| Basic yellow 87 | 0.03 |
| HC Yellow 2 | 0.03 |
| Trimethyl pentaphenyl trisiloxane | 0.85 |
| Almond oil | 0.10 |
| Vitis vinifera extract | 0.10 (dry matter) |
| Lactic acid | q.s. to pH 4.3 |
| Preservative | q.s. |
| Water | to 100 |

### Example 13

| | % by weight |
|---|---|
| Polyquaternium-6 | 0.80 |
| Ceteareth 20 | 1.25 |
| Cetearyl alcohol | 5.00 |
| Behenamidopropyl trimonium chloride | 0.15 |
| Isopropyl palmitate | 0.50 |
| Dimethicone/Dimethiconol | 0.85 |
| Ethylhexyl methoxy cinnamate | 0.10 |
| Lactic acid | q.s. to pH 4.4 |
| Preservative | q.s. |
| Water | to 100 |

## Claims

1. An aqueous composition for human hair **characterized in that** it comprises one or more non-volatile oils liquid at 20°C and at atmospheric pressure and one or more cationic quaternary ammonium polymers having a cationic charge density of 3.0 mEq/g or more as the deposition aid to the one or more oils.

2. The composition according to claim 1 **characterized in that** the cationic quaternary ammonium polymer has a charge density of 3.5 to 8 mEq/g, more preferably 4 to 7.5 mEq/g, most preferably 4.5 to 7.0 mEq/g and in particular 4.5 to 6.5 mEq/g.

3. The composition according to claims 1 and 2 **characterized in that** the cationic quaternary ammonium polymer is selected from Polyquaternium-37 and Polyquaternium-16, preferably it is Polyquaternium-37.

4. The composition according to any one of the claims 1 to 3 **characterized in that** it comprises one or more cationic quaternary ammonium polymers at a total concentration of 0.1 to 5% by weight calculated to the total composition.

5. The composition according to one of the claims 1 to 4 **characterized in that** one or more non-volatile oils is selected from non-volatile silicone oils, natural and synthetic oils, fatty alcohol dialkyl ethers and fatty acid fatty alcohol esters.

6. The composition according to one of the claims 1 to 5 **characterized in that** one or more non-volatile oil is selected from dimethicone, dimethiconol, polydimethylsiloxane (DC fluid ranges from Dow Corning), arylated silicones such as phenyl methicone, phenyl trimethicone, diphenyl dimethicone, diphenylsiloxy phenyl trimethicone, tetramethyl tetraphenyl trisiloxane, triphenyl trimethicone, and trimethyl pentaphenyl trisiloxane, preferably it is dimethiconol or trimethyl pentaphenyl trisiloxane.

7. The composition according to any one of the claims 1 to 7 **characterized in that** it comprises one or more non-volatile oils at a total concentration in the range of 0.1 to 5% by weight calculated to the total composition.

8. The composition according to any one of the claims 1 to 8 **characterized in that** one or more nonionic surfactants are selected from
a- fatty alcohol ethoxylates of the following general structure
R₁ (OCH₂CH₂)ₙ OH
wherein R₁ is straight or branched, saturated or unsaturated alkyl chain which may be synthetic or natural with a C chain length in the range of 8 to 40, preferably 9 to 30 and more preferably 9 to 24 and n is a number in the range of 5 to 40, preferably 9 to 30.
b- polypropylene glycol ether of fatty alcohols according to general structure
R₂(OCH₂(CH₃)CH₂)ₙOH
wherein R₂ is straight or branched, saturated or unsaturated fatty alcohol which may be synthetic or natural with a C chain length in the range of 8 to 40, preferably 9 to 30 and more preferably 9 to 24 and n is a number in the range of 1 to 40, preferably 3 to 30.
c- polyethylene glycol fatty acid esters of the following general structure
R₃ C(O) (OCH₂CH₂)ₙ OH
wherein R₃ is straight or branched, saturated or unsaturated alkyl group which may be synthetic or natural with a C chain length in the range of 7 to 39, preferably 9 to 29 and more preferably 9 to 23 and n is a number in the range of 5 to 40, preferably 9 to 30.
d- polypropylene glycol fatty acid esters of the following general structure
R₄C(O)(OCH₂(CH₃)CH₂)ₙOH
wherein R₄ is straight or branched, saturated or unsaturated alkyl group which may be synthetic or natural with a C chain length in the range of 7 to 39, preferably 9 to 29 and more preferably 9 to 23 and n is a number in the range of 1 to 40, preferably 9 to 30.
e- polyethylene glycol and polypropylene glycol ether of fatty alcohols of the following general structure
R₅(OCH₂(CH₃)CH₂)ₙ₁(OCH₂CH₂)ₙ₂OH
wherein R₅ is straight or branched, saturated or unsaturated alkyl group which may be synthetic or natural with a C chain length in the range of 7 to 39, preferably 9 to 29 and more preferably 9 to 23 and n1 and n2 may be the same or different and are a number in the range of 1 to 40.
f- ethoxylated triglycerides.

9. The composition according to any one of the claims 1 to 9 **characterized in that** it comprises one or more nonionic surfactant at a total concentration of 0.1 to 5%, preferably 0.2 to 4%, more preferably 0.25 to 3% and most preferably 0.3 to 2.5% by weight, calculated to the total composition.

10. The composition according to one of the claims 1 to 10 **characterized in that** it comprises quaternary ammonium surfactant of the general structure
R₁₁ R₁₂ R₁₃ R₁₄ N
wherein R₁₁ is an alky chain having C length of 8 to 30 which may be saturated or unsaturated, straight or branched, R₁₂ an alky chain having C length of 1 to 30 which may be saturated or unsaturated, straight or branched, R₁₁ and R₁₂ additionally may take the structures of
R₁₅ C(O) O (CH2) n or R₁₅ C(O) NH (CH2)n
wherein R15 is an alkyl chain with a C length of 7 to 29 which may be saturated or unsaturated, straight or branched and n is a number between 1 and 4, R₁₃ and R₁₄ is an alkyl chain with a C length of 1 to 4 which may be straight or branched (only for C3 and C4), wherein all alkyl chains may include one or more substituents such as hydroxyl or (poly)ethoxy groups.

11. The composition according to any of one of the proceeding claims **characterized in that** it comprises hair direct dye selected from cationic and neutral nitro dyes.

12. The composition according to any of one of the proceeding claims **characterized in that** it comprises one or more of the following compounds
- Organic solvent,
- Fatty alcohol of the general structure
R₆-OH
wherein R₆ is straight or branched, saturated or unsaturated alkyl chain which may be synthetic or natural with a C chain length in the range of 12 to 24 which may as well be substituted with one or more groups on the alkyl chain,
- UV filter,
- Natural plant extract,
- Protein hydrolyzate,
- Ubiquinone, and
- Nonionic polymer.

13. The composition according to any one of the proceeding claims **characterized in that** it has a pH in the range of 3.0 to 7.0.

14. Method of depositing non-volatile oil onto hair which is liquid at 20°C and at atmospheric pressure wherein a composition according to claims 1 to 14 is applied onto hair and rinsed off from hair after leaving on the hair for a period 1 to 30 min.

15. Method of depositing non-volatile oil onto hair which is liquid at 20°C and at atmospheric pressure wherein a composition according to claims 1 to 14 is applied onto hair and rinsed off from hair after leaving on the hair for a period 1 to 30 min wherein the oil(s) are not washed off from hair completely with single shampooing, preferably not washed off from hair completely with 1 to 5 shampooing cycles.
